# EUROPEAN PATENT APPLICATION

(11) **EP 2 520 662 A1**
(43) Date of publication of application: **07.11.2012**
(21) Application number: 11164798.8
(22) Date of filing: 04.05.2011
(51) Int. Cl.: C12Q 1/68

(54) **Means and methods to determine a risk of multiple organ failure**

(71) Applicant: Stichting Sanquin Bloedvoorziening, 1066 CX Amsterdam (NL)
(72) Inventor: The designation of the inventor has not yet been filed
(74) Representative: Jansen, Cornelis Marinus

(57) **Abstract**

The invention relates to means and methods for typing a sample of an individual to determine a risk of said individual to develop multiple organ failure.

## Description

The invention relates to the fields of biology and medicine, more specifically the invention relates to multiple organ failure.

Multiple organ failure (MOF), also called multiple organ dysfunction syndrome (MODS), is a common cause of death in intensive care units (ICU). MOF is generally defined as the presence of failure in at least two organ systems such that homeostasis of the organ systems can not be maintained or restored without medical intervention. The chance of survival generally reduces with an increasing number of organs involved in MOF. Examples of failure organ systems are failure of the respiratory system, failure of hepatic, renal or gastrointestinal function and circulatory failure.

The common pathophysiology of MOF appears to be a systemic inflammatory response, which can be either sepsis or a condition referred to as systemic inflammatory response syndrome (SIRS). MOF mainly results from infection, tissue ischemia, hypermetabolism and injury, resulting for instance from accidents, burns, radiation injury and surgery.

Treatment of MOF is non-specific and mainly supportive including for instance treatment of infection, nutritional support and artificial support for individual failed organs, such as dialysis and tissue perfusion or oxygenation. Several immunomodulatory interventions, including treatment with immunoglobulin or IFN-Y, have been tested, with a low rate of success.

The development of MOF in a patient is currently established by classification systems such as the KNAUS criteria for Multiple System Organ Failure (Knaus, WA et al. Ann. Surg. 1985; 202:685-293), which involve physiological measurement such as respiratory frequency, heart rate and arterial pressure, urine volume, serum creatinine, and a patient questionnaire, resulting in a score on a scale of 1 to 10. A KNAUS score of 5 or higher is indicative of the presence of MOF. The KNAUS score is determined daily for patients at risk of developing MOF. The use of KNAUS score is not always effective in determining MOF. False positive scores are easily obtained, for instance for patients with artificial respiration. In addition, surgery patients are generally tested positive without MOF being present using the KNAUS score on the first day following surgery. Finally, the KNAUS score can only be used to determine the presence of MOF but not to assess the risk of future development of MOF. Currently no methods are available which enable assessment of the risk of developing MOF, before the first signs of MOF become apparent.

MOF is correlated with prolonged periods in the ICU, which is associated with high costs and a high burden for the patient and a patient's relatives. It is therefore important to prevent MOF, as well as to be able to identify patients at risk of developing MOF early, so that necessary measures can be taken timely.

It is an aim of the present invention to provide methods and means which enable identification of the risk of an individual to develop multiple organ failure. Preferably, means and methods are provided for enabling the prediction of the development of MOF before symptoms of MOF become apparent.

The present invention provides the insight that expression levels of expression products of herein disclosed genes are indicative for a risk of MOF. Such expression levels are different in individuals suffering from a condition or receiving or having received a treatment associated with a risk of MOF, who develop MOF as compared to the expression levels of expression products of these genes in individuals suffering from the same condition or receiving or having received the same treatment, who do not develop MOF. Furthermore, the invention provides the insight that expression levels of expression products of herein disclosed genes in an individual who underwent surgery and develops MOF following said surgery differ from the expression levels of expression products of these genes in said individual before surgery is performed. Thus, by measuring expression levels of at least one gene provided herein, it has become possible to determine a risk of an individual to develop MOF. The herein identified genes are depicted in Table 1 and 2.

The invention therefore provides a method for typing a sample of an individual to determine a risk of said individual to develop multiple organ failure, comprising:
- determining a level of expression of a product of at least one gene selected from Table 1, said level of expression being determined using said sample of said individual;
- comparing said level of expression with a level of expression of a product of said at least one gene of a reference sample, and
- typing said sample based on the levels of expression determined for said at least one gene.

The invention further provides a method for typing a sample of an individual to determine a risk of said individual to develop MOF, comprising:
- determining a level of expression of a product of at least two genes selected from Table 1, said level of expression being determined using said sample of said individual;
- comparing said level of expression with a level of expression of a product of said at least two genes of a reference sample, and
- typing said sample based on the levels of expression determined for said at least two genes,
wherein at least one of said genes is MMP or IL18RAP.

In another embodiment the invention provides a method for typing a sample of an individual to determine a risk of said individual to develop MOF, comprising:
- determining a level of expression of a product of at least one gene selected from Table 2, said level of expression being determined using said sample of said individual;
- comparing said level of expression with a level of expression of a product of said at least one gene of a reference sample, and
- typing said sample based on the levels of expression determined for said at least one gene.

Now that a method for determining a risk of an individual to develop MOF has been provided, it has become possible to provide optimal care for an individual for whom a risk of developing MOF is determined. For instance, such individual can be transferred to an intensive care unit (ICU) before MOF has become apparent, or kept in an ICU after surgery if said individual has not been transferred to a general medical ward yet. Furthermore, if it is known that an individual will develop MOF or is at risk of developing MOF, specific organ function of an individual can be closely monitored for signals of MOF and appropriate supportive care, such as (sedative or analgesic) medication, appropriate nutrition, and family support can be provided. Finally, treatment to prevent or limit MOF can be initiated if it is known that an individual will develop MOF or is at risk of developing MOF. Non-limiting examples of such treatment are immunosuppressive therapy using for instance corticosteroids or specific cytokine therapy, mechanical ventilation and sedative interruption. Preventing or limiting failure of the cardiovascular system can be achieved with use of crystalloid and colloid solutions and blood products.

Also, if it is known that an individual has a low risk of developing MOF, hospital care for such individual can be optimized as well. For instance, if it is known that a risk of developing MOF is low, a patient's stay in an ICU need not be longer than necessary based on the patient's physical condition.

With individually adjusted care for a patient of whom the risk of developing MOF is known, the length of stay in an ICU of said patient can be diminished, which is advantageous both economically and for the well-being of the patient and its relatives. Furthermore, with such individualised optimal care the total length of stay in the medical ICU of individuals suffering from a condition or having undergone a treatment, such as surgery, associated with the development of MOF in a hospital is diminished. Since prolonged periods in an ICU are associated with high costs, this potentially provides a major reduction of costs for a hospital.

With a method of the invention, for any individual having a condition or receiving or having received treatment where there is a risk of developing MOF said risk can be determined, which includes any individual submitted to an ICU of a hospital or any individual who has during hospitalization spent time in an ICU. Non-limiting examples of conditions involving a risk of developing MOF are infection, tissue or organ ischemia, hypermetabolism, and injury, for instance as a result of accidents, burns, radiation, surgery and other major trauma. Examples of treatment where there is a risk of developing MOF include, but are not limited to, surgery, for instance cardiac surgery such as coronary artery surgery and valve replacement surgery, pulmonary surgery, arterial surgery such as endovascular surgery for aortic aneurysms and venous surgery, eye surgery such as cataract surgery and laser eye surgery, orthopaedic surgery such as knee or hip replacement, spinal surgery, neurosurgery, gastro-intestinal surgery, thoracic surgery, organ transplant.

MOF is known to affect individuals suffering from tissue or organ damage due to ischemia in the tissue or organ, for instance reperfusion injury. Ischemia can be caused by a variety of factors, including, but not limited to, blood clots, atherosclerosis, hypotension, surgery, organ removal, such as (partial) liver or kidney removal, and tumours. Therefore, in a preferred embodiment, an individual for whom a risk of developing MOF is determined has suffered from tissue or organ ischemia, such as ischemia in the heart, kidney, liver, lung, large or small intestine, or brain.

MOF is also known to affect patients who underwent surgery, in particular major surgery. Therefore, in another preferred embodiment an individual for whom a risk of developing MOF is determined has undergone surgery. More preferably said individual has undergone major surgery. As used herein, major surgery is defined as any surgery in which the patient receives general anaesthesia and/or is given respiratory supportive therapy by mechanical ventilation assistance. Examples of major surgery as defined herein are cardiac surgery, vascular surgery, organ transplant, gastro-intestinal surgery, thoracic surgery, neurosurgery, spinal surgery, knee or hip replacement.

The genes depicted in Table 1 and 2 were identified in an analysis identifying association between gene expression profiles and MOF of individuals who underwent cardiac-thoracic surgery with cardiopulmonary bypass (CPB). Therefore, a preferred embodiment of a method according to the invention involves using a sample of an individual who underwent cardiac surgery. In another preferred embodiment, a risk of developing MOF is determined using a sample of an individual who underwent cardiopulmonary bypass, for instance during cardiac surgery or pulmonary surgery.

Any sample comprising expression products of genes is, in principle, suitable for a method according to the invention. Preferably, however, said sample is a blood sample, because a blood sample is easily obtained from an individual with little discomfort for said individual. Furthermore, as shown in the Example, the expression levels of gene products in a blood sample are particularly indicative of a risk of developing MOF. Said blood sample preferably comprises whole blood, platelets, peripheral blood mononuclear cells (PBMC's), and/or buffy coat. Most preferably, said sample is a whole blood sample. An expression product of a gene comprises for instance nucleic acid molecules and/or proteins. Preferably said product is isolated from said sample of said individual. A preferred method of the invention comprises using a pre-existing sample from said individual.

Analysis of expression products according to the invention can be performed with any method known in the art. Protein levels are for instance measured using antibody-based binding assays. Enzyme labeled, radioactively labeled or fluorescently labeled antibodies are for instance used for detection and quantification of protein. Assays that are for instance suitable include enzyme-linked immunosorbent assays (ELISA), radio-immuno assays (RIA), Western Blot assays and immunohistochemical staining assays. Alternatively, in order to determine the expression level of multiple proteins simultaneously protein arrays such as antibody-arrays are for instance used.

An expression product according to the invention preferably comprises RNA, such as total RNA or mRNA. RNA is preferred because the lifespan of RNA molecules is shorter than the lifespan of proteins. RNA levels are therefore more representative of the status of an individual at the time of sample preparation, and thus are more suitable for determining a risk of developing MOF in an individual suffering from a condition or receiving or having received treatment associated with a risk of developing MOF. Furthermore, determining RNA expression levels is less laborious than determining protein levels. For instance oligonucleotides arrays are used that are easier to develop and process than protein chips.

Therefore, in a preferred embodiment of the invention a product of a gene according to the invention is RNA, preferably total RNA. Accordingly, the invention provides a method for typing a sample of an individual to determine a risk of said individual to develop MOF, the method comprising determining an RNA expression level of at least one gene selected from Table 1, said RNA being obtained from, and/or present in, a sample of said individual, comparing said RNA expression level with an RNA expression level of said at least one gene in a reference sample, and typing said sample based on the RNA expression levels determined for said at least one gene.

The invention further provides a method for typing a sample of an individual to determine a risk of said individual to develop MOF, the method comprising determining an RNA expression level of at least two genes selected from Table 1, said RNA being obtained from, and/or present in, a sample of said individual, comparing said RNA expression level with an RNA expression level of said at least two gene in a reference sample, and typing said sample based on the RNA expression levels determined for said at least two genes, wherein at least one of said genes is MMP9 or IL18RAP.

In another preferred embodiment, the invention provides a method for typing a sample of an individual to determine a risk of said individual to develop MOF, the method comprising determining an RNA expression level of at least one gene selected from Table 2, said RNA being obtained from, and/or present in, a sample of said individual, comparing said RNA expression level with an RNA expression level of said at least one gene in a reference sample, and typing said sample based on the RNA expression levels determined for said at least one gene.

A reference sample is preferably a sample comprising an expression product of at least one gene selected from Table 1 or 2. In one embodiment, said reference sample comprises an expression product of at least two genes selected from Table 1, wherein at least one of said genes is MMP9 or IL18RAP. Said reference sample is preferably the same type of sample as a sample of an individual for whom a risk of developing MOF is determined. For instance, if said sample of said individual for whom a risk of developing MOF is determined is a whole blood sample, said reference sample is preferably a whole blood sample as well. Furthermore, a product of a gene of a reference sample is preferable the same type of product as a product of said gene in a sample of an individual for whom a risk of developing MOF is determined. For instance, if said product of said gene of said sample of said individual for whom a risk of developing MOF is determined is RNA, said product of said gene of said reference sample is preferably RNA as well. In a preferred embodiment a reference sample comprises RNA, more preferably total RNA.

As described herein before, the present inventors found that the expression of the genes depicted in Table 1 and 2 differs between individuals suffering from a condition or receiving or having received a treatment associated with a risk of developing MOF who will develop MOF and individuals suffering from the same condition or having received the same treatment who did not develop MOF. Thus, a reference sample preferably comprises at least one sample from an individual who did not develop MOF. Said reference sample preferably comprises pooled samples of individuals who did not develop MOF.

Therefore, in a preferred embodiment an individual for whom a risk of developing MOF is determined is suffering from a condition or receiving or has received a treatment associated with a risk of developing MOF, and a reference sample comprises pooled samples of individuals suffering from the same condition or having received the same treatment who did not develop MOF. As used herein the term "pooled samples of individuals who did not develop MOF" indicates that a reference sample comprises an expression product of at least one gene selected from Table 1 or 2 of at least two individuals who did not develop MOF. Preferably said reference sample comprises an expression product of a gene or genes of at least five individuals who did not develop MOF, more preferably of at least ten individuals who did not develop MOF, more preferably of at least fifteen individuals who did not develop MOF, most preferably of at least twenty individuals who did not develop MOF. As indicated, said individual or individuals who did not develop MOF are preferably suffering from a condition or receiving or having received treatment associated with a risk at developing MOF, preferably the same condition or treatment as an individual for whom a risk of developing MOF is determined. As used herein, a "condition or treatment associated with a risk of developing MOF" is defined as any condition or treatment of which it is known that MOF can develop within a month of the occurrence or initiation of said condition or treatment in an individual. Preferably, MOF is known to develop within twenty days, more preferably ten days, more preferably five days, after the occurrence or initiation of said condition or treatment. Such condition or treatment associated with a risk of developing MOF includes, but is not limited to, infection, tissue or organ ischemia, hypermetabolism, and injury, for instance as a result of accidents, burns, radiation and surgery. For instance, if an individual for whom a risk of developing MOF is determined or is to be determined has undergone cardiac surgery, a reference sample preferably comprises at least one sample, preferably pooled samples, of at least one individual, preferably at least two individuals, who also has undergone cardiac surgery, but who did not develop MOF following said surgery.

The inventors further found that the expression level of a product of genes depicted in Table 1 and 2 also differs to a larger extent before and after surgery within an individual who will develop MOF, as compared to the difference of said expression level before and after surgery within an individual who will not develop MOF. Therefore, in another preferred embodiment, a sample of an individual for whom a risk of developing MOF is determined is from after said individual has undergone treatment associated with a risk of developing MOF, such as surgery, preferably major surgery, and a reference sample is from the same individual prior to said treatment. Said reference sample can be obtained from said individual at any time prior to surgery. However, preferably said reference sample is obtained from said individual within one week prior to surgery, preferably within six days prior to surgery, more preferably within five days prior to surgery, more preferably within four days prior to surgery, more preferably within three days prior to surgery, such as three days before surgery, within two days before surgery, most preferably within one day before surgery. This way, the influence of other variables, such as age, on the expression of gene products is limited. Optionally said reference sample is obtained from said individual shortly before surgery, such as 30 minutes, one hour or two hours before surgery. If MOF develops in an individual who has undergone surgery, MOF is generally present three to four days after said surgery has been performed. Therefore, said sample of said individual obtained after surgery is preferably obtained within two days after surgery. This way, a sufficient amount of time is present between obtaining said sample from said individual and the development of MOF, so that necessary measures can be initiated before the symptoms of MOF are present. Preferably said sample is obtained within one day after surgery, after said individual has been removed from respiratory assistance and has recovered from anaesthesia. As used herein, the term "after surgery" indicates after said surgery has finished. The sooner a sample is obtained after surgery, the more time is available to take the necessary measures.

Table 1 shows the genes which in a whole blood sample of individuals who developed MOF following cardiac surgery have an at least 2 fold higher or lower expression level than said genes in a whole blood sample of individuals who did not develop MOF following cardiac surgery. The samples were obtained approximately 24 hours after surgery, thus before the development of MOF was apparent. A positive fold-change indicates that the expression level of a gene is higher in individuals who develop MOF after surgery as compared to the expression level in individuals who do not develop MOF after surgery. A negative fold-change indicates that the expression level of a gene is lower in individuals who develop MOF after surgery as compared to the expression level in individuals who do not develop MOF after surgery.

In one embodiment, it is preferred that at least one gene selected from Table 1 or 2 is a gene that is upregulated in individuals who develop MOF as compared to individuals who did not develop MOF. Said upregulated gene is, or said upregulated genes are, preferably selected from C19orf59, MMP9, ARG1, IL1R2, ZDHHC19, PFKFB3, IL18RAP, S100A12, CD55, FKBP5, CA4, ALPL, LILRA5, CDK5RAP2, RETN, OLFM4, C5orf32, LOC153561, MFGE8, GADD45A, CD163 and MX1, or a combination thereof. In a particularly preferred embodiment, said upregulated gene is, or said upregulated genes are, selected from ZDHHC19, MMP9 and IL18RAP, or a combination thereof. Most preferably, expression levels of products of ZDHHC19, MMP9 and IL18RAP are determined. As is described in more detail hereafter, using these genes a sensitivity for the prediction of a risk of developing MOF of 100% was obtained. A positive fold change of at least two in the level of expression of said genes in an individual as compared to the level of expression of said genes of a reference sample is indicative of the presence of a risk of said individual to develop MOF. In another embodiment, it is preferred that at least one gene selected from Table 1 or 2 is a gene that is downregulated in individuals who will develop MOF, as compared to individuals who did not develop MOF. Said downregulated gene is , or said downregulated genes are, preferably selected from CLC, PTGS2, IL8 and CX3CR1 or a combination thereof. A negative fold change of at least two in the level of expression of at least one of said genes, preferably at least two of said genes, in an individual as compared to the level of expression of said gene of a reference sample is indicative of the presence of a risk of said individual to develop MOF.

From each gene listed in Tables 1 and 2 it has been determined whether said gene has an at least 2-fold higher or lower expression level in individuals suffering from a condition or receiving or having received a treatment associated with the risk of developing MOF who will develop MOF as compared to individuals suffering from the same condition or having received the same treatment who will not develop MOF. When determining levels of expression products of a set of genes according to the invention using samples from individuals suffering from such condition or having received such treatment, not all of said genes will always be regulated as indicated in Tables 1 and 2. It is possible that certain individuals at risk of developing MOF will have an expression level of one or several genes according to the invention that does not match with the differential expression pattern indicated in Tables 1 and 2. However, the overall expression pattern of an individual who will develop MOF will still resemble the differential expression pattern indicated in Table 1 or 2 more than the expression pattern of individuals who will not develop MOF. Typing of a sample from an individual with a method according to the invention therefore in one embodiment comprises determining a level of expression of a product of at least two genes that are selected from Table 1 or 2. In other embodiments, a level of expression of a product of at least three genes that are selected from Table 1 or 2, at least four genes that are selected from Table 1 or 2, at least five genes that are selected from Table 1 or 2, at least six genes that are selected from Table 1 or 2, at least seven genes that are selected from Table 1 or 2, at least eight genes that are selected from Table 1 or 2, at least nine genes that are selected from Table 1 or 2, or at least ten genes that are selected from Table 1 or 2 are determined. In further embodiments, a level of expression of a product of at least fifteen genes that are selected from Table 1 or 2, at least twenty genes that are selected from Table 1 or 2, or at least 25 genes that are selected from Table 1 or 2 are determined. In a further embodiment, a method of the invention comprises determining a level of expression of a product of all genes of Table 1 or all genes of Table 2. Generally, determining the expression levels of multiple genes enables the determination of a risk of developing MOF more accurately. Preferably, said product is RNA. Preferably, a level of expression of a product of at least ZDHHC19, MMP9 and IL18RAP is determined.

Thus in order to type a sample with a method according to the invention, it is preferably determined whether up- or downregulation of a set of genes resembles up- or downregulation of said genes as indicated in Table 1 or 2. If the expression levels of a set of genes according to the invention of a sample of an individual resemble the expression levels of said genes as indicated in Table 1 or 2, the sample is typed as said individual being at risk of developing MOF. If, on the other hand, the expression levels of said set of genes does not resemble the expression levels of said genes as indicated in Table 1 or 2, the tested sample is typed as said individual having a low risk of developing MOF.

As shown in the Example, the present inventors succeeded in predicting the development of MOF in individuals after surgery, with a sensitivity of 100% by determining the levels of ZDHHC19, MMP9 and/or IL18RAP RNA expression using a sample of said individuals before and after surgery. Thus, with a method according to the invention it has become possible to identify all individuals that will develop MOF after surgery. Since MOF is a leading cause of death of patients in ICU, a method which enables prediction of the development of MOF with a sensitivity of 100% is highly desirable.

Therefore, in a preferred embodiment, a method of the invention comprises determining a level of expression of a product of at least one gene selected from Table 1 or 2 using a sample of an individual after surgery and using a reference sample from said individual prior to surgery. In another preferred embodiment, a method of the invention comprises determining a level of expression of a product of at least two genes selected from Table 1, wherein at least one of said genes is MMP9 or IL18RAP using a sample of an individual after surgery and using a reference sample from said individual prior to surgery. Preferably said surgery is major surgery, more preferably cardiac surgery. In a preferred embodiment a level of expression of a product of at least three genes selected from Table 1, wherein at least one of said genes is MMP9 or IL18RAP, is determined. The at least three genes selected from Table 1 are preferably selected from ZDHHC19, MMP9 and IL18RAP, or a combination thereof.

In a preferred embodiment, a method of the invention comprises determining a level of ZDHHC19 (Zinc finger DHHC-type containing19) RNA expression, preferably in combination with determining a level of MMP9 and/or IL18RAP expression. Preferably it is determined whether a fold change in the level of expression of ZDHHC19 using a sample of an individual after surgery is at least 42 as compared to the level of expression of ZDHHC19 of a reference sample of said individual prior to surgery because according to the present invention this is indicative of the presence of a risk of said individual to develop MOF. More preferably, it is determined whether a fold change in the level of expression of ZDHHC19 in a sample of an individual is at least 50, more preferably at least 60, more preferably at least 65, more preferably at least 70, more preferably at least 75, more preferably at least 80, more preferably at least 85, more preferably at least 90, more preferably at least 93, as compared to the level of expression of ZDHHC19 of a reference sample. Such expression levels are even more indicative of the presence of a risk of said individual to develop MOF. Preferably it is determined whether a fold change in the level of expression of ZDHHC19 using a sample of an individual after surgery is between 42 and 300 as compared to the level of expression of ZDHHC19 of a reference sample of said individual prior to surgery because this is particularly indicative of the presence of a risk of said individual to develop MOF. More preferably, it is determined whether a fold change in the level of expression of ZDHHC19 using a sample of an individual is between 42 and 290, more preferably between 42 and 280, more preferably between 42 and 270, more preferably between 42 and 269, as compared to the level of expression of ZDHHC19 of a reference sample.

In a more preferred embodiment, it is determined whether a fold change in the level of expression of ZDHHC19 using said sample of said individual after surgery is between 50 and 300 as compared to the level of expression of ZDHHC19 of a reference sample of said individual prior to surgery. Preferably it is determined whether a fold change in the level of expression of ZDHHC19 using a sample of said individual is between 60 and 290, more preferably between 70 and 280, more preferably between 80 and 270, more preferably between 90 and 270, as compared to the level of expression of ZDHHC19 of said reference sample. In a particularly preferred embodiment it is determined whether a fold change in the level of expression of ZDHHC19 using said sample of said individual of between 93 and 269 as compared to the level of expression of ZDHHC19 of said reference sample.

In another preferred embodiment, a method of the invention comprises determining a level of IL18RAP (Interleukin-18 receptor associated protein) RNA expression. Preferably it is determined whether a fold change in the level of RNA expression of IL18RAP using a sample of an individual after surgery is at least 8.5 as compared to the level of RNA expression of IL18RAP of a reference sample of said individual prior to surgery because according to the invention this is indicative of the presence of a risk of said individual to develop MOF. More preferably, it is determined whether a fold change in the level of RNA expression of IL18RAP using said sample of said individual after surgery is at least 10, more preferably at least 12, more preferably at least 14, more preferably at least 16, more preferably at least 18, more preferably at least 20, more preferably at least 22, more preferably at least 23, more preferably at least 23.5, as compared to the level of RNA expression of IL18RAP of said reference sample. Preferably it is determined whether a fold change in the level of RNA expression of IL18RAP using a sample of an individual after surgery is between 8.5 and 60 as compared to the level of RNA expression of IL18RAP of a reference sample of said individual prior to surgery because this is even more indicative of the presence of a risk of said individual to develop MOF. More preferably, it is determined whether a fold change in the level of RNA expression of IL18RAP using said sample of said individual is between 8.5 and 50, more preferably between 8.5 and 45, more preferably between 8.5 and 40, more preferably between 8.5 and 36, as compared to the level of RNA expression of IL18RAP of said reference sample.

In a more preferred embodiment, it is determined whether a fold change in the level of RNA expression of IL18RAP using said sample of said individual is between 10 and 60 as compared to the level of RNA expression of IL18RAP of said reference sample. Preferably, it is determined whether a fold change in the level of RNA expression of IL18RAP in a sample of an individual is between 12 and 50, more preferably between 15 and 45, more preferably between 20 and 40, as compared to the level of RNA expression of IL18RAP in a reference sample. In a particularly preferred embodiment it is determined whether a fold change in the level of RNA expression of IL18RAP using said sample of said individual of between 23.5 and 36 as compared to the level of RNA expression of IL18RAP of said reference sample.

In another preferred embodiment, a method of the invention comprises determining a level of MMP9 (Matrix metallopeptidase 9) RNA expression. Preferably it is determined whether a fold change in the level of RNA expression of MMP9 using a sample of an individual after surgery is at least 10 as compared to the level of RNA expression of MMP9 of a reference sample of said individual prior to surgery because according to the invention this is indicative of the presence of a risk of said individual to develop MOF. More preferably, it is determined whether a fold change in the level of RNA expression of MMP9 using said sample of said individual is at least 11, more preferably at least 12, more preferably at least 13, more preferably at least 14, more preferably at least 14.8, as compared to the level of RNA expression of MMP9 of said reference sample. Preferably it is determined whether a fold change in the level of RNA expression of MMP9 using a sample of an individual after surgery is between 10 and 50 as compared to the level of RNA expression of MMP9 of a reference sample of said individual prior to surgery because this is even more indicative of the presence of a risk of said individual to develop MOF. More preferably, it is determined whether a fold change in the level of RNA expression of MMP9 using said sample of said individual is between 10 and 40, more preferably between 10 and 35, more preferably between 10 and 30, more preferably between 10 and 26.1, as compared to the level of RNA expression of MMP9 of said reference sample.

In a more preferred embodiment, it is determined whether a fold change in the level of RNA expression of MMP9 using said sample of said individual is between 11 and 50 as compared to the level of RNA expression of MMP9 of said reference sample. Preferably, it is determined whether a fold change in the level of RNA expression of MMP9 using a sample of an individual is between 12 and 40, more preferably between 13 and 35, more preferably between 14 and 30, as compared to the level of RNA expression of MMP9 of a reference sample. In a particularly preferred embodiment it is determined whether a fold change in the level of RNA expression of MMP9 using said sample of said individual is between 14.8 and 26.1 as compared to the level of RNA expression of MMP9 of said reference sample is indicative of the presence of a risk of said individual to develop MOF.

In a particularly preferred embodiment, it is determined whether a fold change of ZDHHC19, MMP9 and IL18RAP RNA expression are at least the above indicated levels or within the above indicated ranges. As indicated herein before, by determining the expression levels of these three genes a sensitivity for predicting a risk of developing MOF of 100% is achieved. As demonstrated in the Example, determining an expression profile according to the invention wherein a fold change in the level of RNA expression of MMP9 is at least 14.8, a fold change in the level of RNA expression of IL18RAP is at least 8.8, or a fold change in the level of RNA expression of ZDHHC19 is between 45 and 93 allows predicting a risk of developing MOF with a specificity of 67%. Thus, only 33% of the individuals who do not develop MOF will be predicted to develop MOF. In one embodiment, a method according to the invention therefore comprises determining whether a fold change in the level of expression of ZDHHC19 in said sample of said individual is between 45 and 93 as compared to the level of expression of ZDHHC19 in said reference sample, and/or a fold change in the level of expression of IL18RAP in said sample of said individual is at least 8.8 as compared the level of expression of IL18RAP in said reference sample, and/or a fold change in the level of expression of MMP9 in said sample of said individual is at least 14.8 as compared to the level of expression of MMP9 in said reference sample, which is indicative of the presence of a risk of said individual to develop multiple organ failure. Preferably, a method comprises determining whether a fold change in the RNA expression level of MMP9, IL18RAP and ZDHHC19 is as indicated above.

Determining an expression profile according to the invention wherein a fold change in the level of RNA expression of MMP9 is between 14.8 and 26.1, a fold change in the level of RNA expression of IL18RAP is between 23.5 and 36, or a fold change in the level of RNA expression of ZDHHC19 is between 93 and 269 even allows predicting a risk of developing MOF with a specificity of 79%. Using this expression profile according to the invention, only a low number of individuals who do not develop MOF, 21%, will be predicted to develop MOF. In a preferred embodiment, a method according to the invention therefore comprises determining whether a fold change in the level of expression of ZDHHC19 in said sample of said individual is between 93 and 269 as compared to the level of expression of ZDHHC19 in said reference sample, and/or a fold change in the level of expression of IL18RAP in said sample of said individual is between 23.5 and 36 as compared the level of expression of IL18RAP in said reference sample, and/or a fold change in the level of expression of MMP9 in said sample of said individual is between 14.8 and 26.1 as compared to the level of expression of MMP9 in said reference sample, which is indicative of the presence of a risk of said individual to develop multiple organ failure. More preferably, a method comprises determining whether a fold change in the RNA expression level of MMP9, IL18RAP and ZDHHC19 is as indicated above.

As indicated above, a sample of an individual for whom a risk of developing MOF is determined or is to be determined preferably comprises RNA, most preferably total RNA. A sample from a individual or a reference sample comprising RNA can be obtained and processed in numerous ways, as is known to a person skilled in the art. For example, such sample can be freshly prepared from cells, blood or tissue at the moment of harvesting, or they can be prepared from samples that are stored frozen, for instance at -70°C, until processed for sample preparation. Cell, blood or tissue samples can also be stored under alternative conditions that preserve the quality of the RNA. Examples of these preservative conditions are fixation using e.g. PAXgene RNA stabilization medium, formaline, RNase inhibitors such as RNAsin (Pharmingen) or RNasecure (Ambion), aquous solutions such as RNAlater (Assuragen), Hepes-Glutamic acid buffer mediated Organic solvent Protection Effect (HOPE), and RCL2 (Alphelys), and non-aquous solutions such as Universal Molecular Fixative (Sakura Finetek USA Inc.). Usually, a chaotropic nucleic acid isolation lysis buffer (Boom method, Boom et al. J Clin Microbiol. 1990; 28:495-503) is used for RNA isolation.

With a method according to the invention, it can be determined quantitatively whether an expression level of an expression product of a gene is higher or lower than the expression level of said expression product in a reference sample. RNA expression levels are, however, preferably quantified. The RNA level of a gene according to the invention can be determined by any method known in the art. RNA levels can for instance be quantified in relation to a household gene and/or can be determined relatively in relation with RNA levels of other genes measured at the same time. Methods to determine RNA levels of genes are known to a skilled person and include, but are not limited to, Northern blotting, (quantitative) PCR, and microarray analysis. Preferred methods for determining a level of RNA expression is quantitative PCR and microarray analysis.

Northern blotting comprises the quantification of RNA of a specific gene by hybridizing a labeled probe that specifically interacts with said RNA, after separation of RNA by gel electrophoresis. Probes are for instance labeled with radioactive isotopes or chemiluminescent substrates. Quantification of the labeled probe that has interacted with said nucleic acid expression product serves as a measure for determining the level of expression. The determined level of expression can be normalized for differences in the total amounts of nucleic acid expression products between two separate samples with for instance an internal or external calibrator by comparing the level of expression of a gene that is known not to differ in expression level between samples or by adding a known quantity of RNA before determining the expression levels.

Additionally or alternatively, RNA is reverse transcribed into cDNA. Reverse transcriptase polymerase chain reaction (RT-PCR) is for instance performed using specific primers that hybridize to an RNA sequence of interest and a reverse transcriptase enzyme. Furthermore, RT-PCR can be performed with random primers, such as for instance random hexamers or decamers which hybridize randomly along the RNA, or oligo d(T) which hybridizes to the poly(A) tail of mRNA, and reverse transcriptase enzyme.

Quantitative Polymerase Chain Reaction (qPCR) provides an alternative method to quantify the level of expression of nucleic acids. qPCR can be performed by real-time PCR (rtPCR), in which the amount of product is monitored during the amplification reaction, or by end-point measurements, in which the amount of a final product is determined. As is known to a skilled person, rtPCR is for instance performed by the use of a nucleic acid intercalator, such as for example ethidium bromide or SYBR® Green I dye, which interacts which all generated double stranded products resulting in an increase in fluorescence during amplification, or for instance by the use of labeled probes that react specifically with the generated double stranded product of the gene of interest. Alternative detection methods that can be used are provided by amongst other things dendrimer signal amplification, hybridization signal amplification, and molecular beacons.

Microarray analysis involves the use of selected nucleic acid molecules that are immobilized on a surface. These nucleic acid molecules, termed probes, are able to hybridize to nucleic acid expression products. The probes are preferably exposed to labeled sample nucleic acid, hybridized, washed and the (relative) amount of nucleic acid expression products in the sample that are complementary to a probe is determined. Microarray analysis allows simultaneous determination of nucleic acid expression levels of a large number of genes. Background correction can be performed for instance according to the "offset" method that avoids negative intensity values after background subtraction. Furthermore, normalization can be performed in order to make the two channels on each single array comparable for instance using global loess normalization, and scale normalization which ensures that the log-ratios are scaled to have the same median-absolute-deviation (MAD) across arrays.

Preferably, if a level of RNA expression is determined by quantitative PCR or microarray analysis, systemic bias is reduced. Systemic bias results in variation of different measurements of RNA expression, and can be due to, for example, inconsistencies in sample handling, plate or array fabrication, staining and scanning. Reducing systemic bias is for instance accomplished by normalization. This involves the detection of RNA expression levels of so-called normalization genes, for instance housekeeping genes such as glyceraldehyde-3-phosphate dehydrogenase (GAPDH), hypoxanthine phosphoribosyltransferase (HPRT) and 18S RNA levels, of which the RNA level is thought to be constant in a given cell or tissue and independent from the disease or treatment affecting said cell or tissue or inflammatory status of said cell or tissue. Another preferred method to reduce systemic bias is to determine the levels of RNA expression using a sample from an individual and a reference sample at the same time, preferably on the same plate which is used to perform quantitative PCR or microarray analysis. This way, variation between the sample and the reference sample due to for instance sample handling, plate or array fabrication and staining is prevented. Additionally, the levels of RNA expression of control samples, for instance blood samples from healthy volunteers, can be simultaneously determined on the same plate or array to determine variation within said plate or array.

The invention further provides a kit of parts comprising at least two gene-specific primers selected from the group consisting of a ZDHHC19-specific primer, an MMP9-specific primer and an IL18RAP-specific primer, and/or at least two probes selected from the group consisting of a ZDHHC19-specific probe, an MMP9-specific probe and an IL18RAP-specific probe. Such kit of parts is particularly useful for determining a risk of an individual to develop multiple organ failure with a method according to the invention, whereby an expression level of ZDHHC19, MMP9 and/or IL18RAP RNA in a sample of said individual are determined. A probe is for instance useful in determining an expression level of ZDHHC19, MMP9 and/or IL18RAP RNA in a sample of said individual using qPCR. A primer pair as used herein is defined as a pair of primers consisting of one forward primer and one reverse primer. At least one primer of said primer pair is specific for ZDHHC19, MMP9 or IL18RAP. The other primer of said primer pair can be either specific for said gene as well or be can be an aspecific primer. An aspecific primer as used herein is defined as a primer which is capable of annealing to a nucleic acid sequence of any gene. A non-limiting example of such an aspecific primer is an oligo(dt) aspecific primer. In a preferred embodiment, a kit of parts comprises a ZDHHC19-specific primer, an MMP9-specific primer and an IL18RAP-specific primer.

In a preferred embodiment, a kit of parts according to the invention comprises a primer pair consisting of a forward primer and a reverse primer specific for ZDHHC19, a primer pair consisting of a forward primer and a reverse primer specific for MMP9, and/or a primer pair consisting of a forward primer and a reverse primer specific for IL18RAP. As used herein the terms "specific primer" and "primer specific for" indicate that said primer preferentially binds to a nucleic acid sequence of the gene said primer is specific for as compared to any other gene. The primers of a primer pair should be located on the gene sequences in such a way that amplification of a nucleic acid product is possible. Therefore, the primers of a primer pair are preferably located within 1000 nucleotides from each other. A nucleic acid product obtained using a primer pair of a kit of part according to the invention in an amplification reaction preferably has a length of between 10 and 1000 nucleotides, more preferably of between 20 and 900 nucleotides, more preferably of between 30 and 800 nucleotides, more preferably of between 40 and 700 nucleotides, most preferably of between 50 and 600 nucleotides.

In another preferred embodiment a kit of parts comprises a ZDHHC19-specific probe, an MMP9-specific probe and an IL18RAP-specific probe. In a particularly preferred embodiment, a kit of parts comprises a ZDHHC19-specific primer, an MMP9-specific primer, an IL18RAP-specific primer, a ZDHHC19-specific probe, an MMP9-specific probe and an IL18RAP-specific probe.

Non-limiting examples of suitable ZDHHC19-specific primers and a suitable ZDHHC19-specific probe are the primers and probe of Applied Biosystems Assay kit Hs00376118-m1. Non-limiting examples of suitable MMP9-specific primers and a suitable MMP9-specific probe are the primers and probe of Applied Biosystems Assay kit Hs00957562-m1. Non-limiting examples of suitable IL18RAP-specific primers and a suitable IL18RAP-specific probe are the primers and probe of Applied Biosystems Assay kit Hs00187256-ml.

A kit of parts according to the invention may further comprise constituents such as, but not limited to, a mixture of nucleotides, DNA polymerase, a suitable buffer for performing an amplification reaction, a means for detecting the presence of amplified nucleic acid, such as a nucleic acid intercalator, or a combination thereof.

A kit of parts according to the invention is particularly suitable for typing a sample of an individual to determine a risk of said individual to develop MOF. Also provided is therefore a use of a kit according to the invention for typing a sample of an individual to determine a risk of said individual to develop MOF.

The invention further provides a use of an MMP9-specific primer or an IL18RAP-specific primer for typing a sample of an individual to determine a risk of said individual to develop multiple organ failure. Preferably at least two primer pairs are used that are selected from the group consisting of a primers pair comprising an MMP9-specific primer, a primer pair comprising a ZDHHC19-specific primer and a primer pair comprising an IL18RAP-specific primer. In a preferred embodiment, at least two ZDHHC19-specific primers, at least two MMP9-specific primers and/or at least two IL18RAP-specific primers are used. In another preferred embodiment, an aspecific primer is used in combination with a ZDHHC19-specific primer, an MMP9-specific primer and/or an IL18RAP-specific primer.

Also provided is a use of an MMP9-specific probe or an IL18RAP-specific probe for typing a sample of an individual to determine a risk of said individual to develop multiple organ failure. Preferably at least two probes are used that are selected from the group consisting of an MMP9-specific probe, a ZDHHC19-specific probe and an IL18RAP-specific probe. In a preferred embodiment, an MMP9-specific probe, a ZDHHC19-specific probe and an IL18RAP-specific probe are used.

A method according to the invention preferably further comprises determining a strategy for treatment of the individual in order to reduce or prevent the symptoms of MOF. Treatment may include, but is not limited to, for example, providing supportive care, medication such as analgesic or sedative medication, immunosuppressive therapy, for instance corticosteroid or cytokine treatment, mechanical ventilation, treatment with crystalloid and colloid solutions or blood products, or any combination thereof.

Further provided by the invention is therefore a method of assigning treatment to an individual who had surgery or is suffering from trauma, comprising typing a sample from the patient, preferably a blood sample, according to a method of the invention, classifying said sample as a risk to developing multiple organ failure or as no risk to developing multiple organ failure, and assigning treatment to an individual of which the sample is classified as a risk to developing multiple organ failure.

The invention is further explained in the following example. This example does not limit the scope of the invention, but merely serves to clarify the invention. Table 1. Transcripts displaying a more then 2-fold differential expression between MOF and no MOF in whole blood.

MOF effect: fold change of individuals that underwent cardiac surgery without blood transfusion who developed MOF compared to individuals that underwent cardiac surgery without blood transfusion who did not develop MOF.

MOF effect (+BT): fold change of individuals that underwent cardiac surgery with blood transfusion (BT) who developed MOF compared to individuals that underwent cardiac surgery with blood transfusion who did not develop MOF.

| **Gene** | **GenBank accession nr. (mRNA)** | **Fold change MOF effect** | **Fold change MOF effect (+BT)** |
|---|---|---|---|
| C19orf59 | NM_174918 | 6,8 | |
| MMP9 | NM_004994 | 6,7 | |
| ARG1 | NM_000045 | 5,5 | |
| IL1R2 | NM_004633 | 4,9 | |
| ZDHHC19 | NM_001039617 | 4,6 | |
| PFKFB3 | NM_004566 | 3,9 | |
| IL18RAP | NM_003853 | 3,8 | |
| S100A12 | NM_005621 | 3,6 | |
| CD55 | NM_000574 | 2,4 | |
| FKBP5 | NM_004117 | 2,3 | |
| CA4 | NM_000717 | 2.2 | |
| ALPL | NM_000478 | 2,1 | |
| LILRA5 | NM_021250 | 2.0 | |
| CDK5RAP2 | NM_018249 | | 4,9 |
| RETN | NM_020415 | | 2,9 |
| OLFM4 | NM_006418 | | 3,2 |
| C5orf32 | NM_032412 | | 4,5 |
| LOC153561 | BC051369 | | 3,4 |
| MFGE8 | NM_005928 | | 2,6 |
| GADD45A | NM_001924 | | 2,4 |
| CD163 | NM_004244 | | 3,9 |
| MX1 | NM_001144925 | 2.2 | |
| CLC | NM_001828 | -5,8 | |
| PTGS2 | NM_000963 | -3,2 | |
| IL8 | NM_000584 | -2,1 | |
| CX3CR1 | NM_001171174 | | -3,5 |

Table 2. Selection of transcripts displaying a more then 2-fold differential expression between MOF and no MOF in whole blood.

MOF effect: fold change of individuals that underwent cardiac surgery without blood transfusion who developed MOF compared to individuals that underwent cardiac surgery without blood transfusion who did not develop MOF.

MOF effect (+BT): fold change of individuals that underwent cardiac surgery with blood transfusion (BT) who developed MOF compared to individuals that underwent cardiac surgery with blood transfusion who did not develop MOF.

| **Gene** | **GenBank accession nr. (mRNA)** | **Fold change MOF effect** | **Fold change MOF effect (+BT)** |
|---|---|---|---|
| MMP9 | NM_004994 | 6,7 | |
| ARG1 | NM_000045 | 5,5 | |
| PFKFB3 | NM_004566 | 3,9 | |
| IL18RAP | NM_003853 | 3,8 | |
| S100A12 | NM_005621 | 3,6 | |
| CD55 | NM_000574 | 2,4 | |
| FKBP5 | NM_004117 | 2,3 | |
| CA4 | NM_000717 | 2.2 | |
| ALPL | NM_000478 | 2,1 | |
| LILRA5 | NM_021250 | 2.0 | |
| CDK5RAP2 | NM_018249 | | 4,9 |
| RETN | NM_020415 | | 2,9 |
| OLFM4 | NM_006418 | | 3,2 |
| C5orf32 | NM_032412 | | 4,5 |
| LOC153561 | BC051369 | | 3,4 |
| MFGE8 | NM_005928 | | 2,6 |
| GADD45A | NM_001924 | | 2,4 |
| CD163 | NM_004244 | | 3,9 |
| MX1 | NM_001144925 | 2.2 | |
| PTGS2 | NM_000963 | -3,2 | |
| IL8 | NM_000584 | -2,1 | |
| CX3CR1 | NM_001171174 | | -3,5 |

### Brief description of the drawing

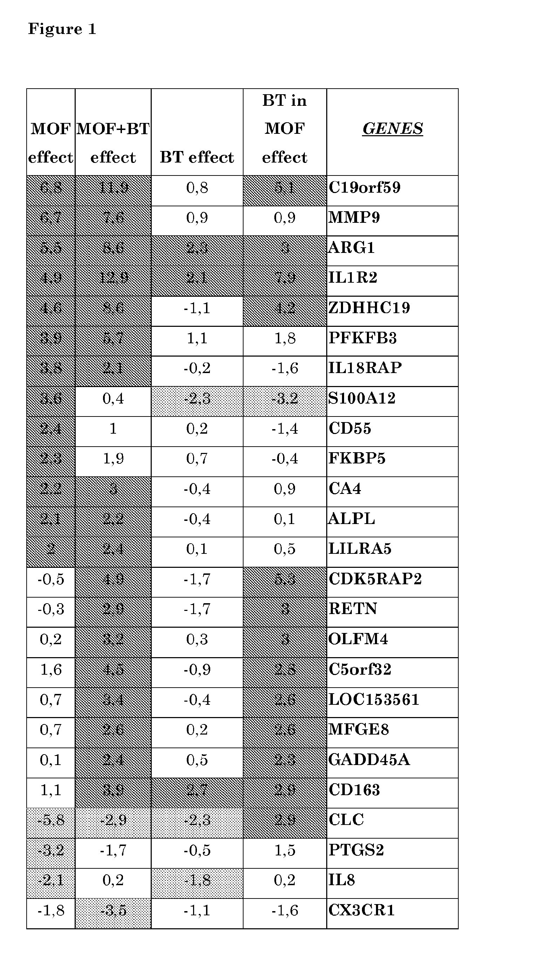

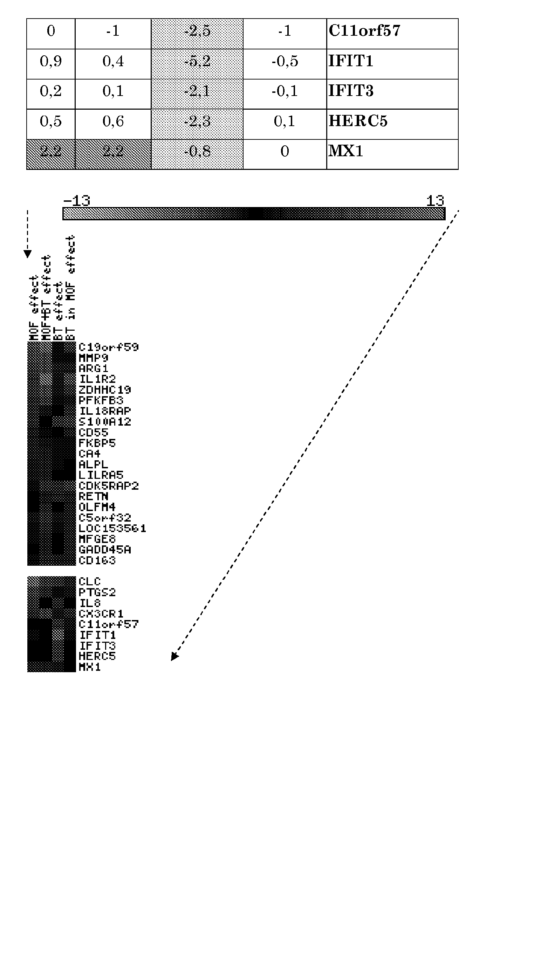

Figure 1. Transcriptome response ranking of 30 genes on > 2 fold change difference between the 4 groups revealing > 2 fold up- or downregulation (AGE) after surgery. The normalized GE (gene expression) responses are presented as numbers and corresponding intensity in the figure in the following order: 13 upregulated and 9 downregulated genes were first ranked on > 2 fold differences between the MOF and no MOF patients without transfusion, and presented as column "MOF effect" and as the corresponding ΔGE responses in the three other columns. Second, 7 upregulated genes were ranked on difference between the transfused and non-transfused MOF patients as presented as column "BT in MOF effect" and as the corresponding ΔGE responses in the three other columns. Third, upregulated CD 163 transcriptome show > 2 fold difference between the transfused and non transfused of no MOF patients as presented as column "BT effect" and as the corresponding ΔGE responses in the three other columns. Fourth, the corresponding transcriptome response differences between the transfused MOF and transfused no MOF patients are shown in column "MOF+BT effect" and as the corresponding ΔGE responses in the three other columns.

Light grey is > 2 fold decreased difference in transcriptome responses between the corresponding groups, dark grey is > 2 fold increased difference in transcriptome responses as compared to the corresponding group. Figure 1 was constructed by matrix2png 1.0.7 (Grisoni ML et al. J Biol Chem 1999; 274: 21491-21494).

### Example

### Materials and methods:

### Patients

For this observational study, one nested case-control of 29 patients and a confirmation cohort of 21 patients were selected from 298 patients who were included in a randomised controlled trial (RCT) (Later AF, et al. Eur.J.Cardiothorac.Surg. 2009; 36:322-329). In this RCT, two different antifibrinolytic drugs were tested (aprotinin and tranexamic acid) against placebo, on reduction of blood loss and transfusion requirement. The trial protocol was approved by the Internal Review board of the Leiden University Medical Centre (LUMC). 234 patients consented to participate in GE research, from which 188 patient's blood was sampled. This encompassed blood sampling prior to and 24 hours after surgery. Thirty out of 234 patients developed MOF as assessed by the criteria described by Knaus et al. (Knaus WA, et al. Ann.Surg. 1985; 202:685-693). Five of these patients were excluded because they had received only FFP, but no RBC transfusions. Eleven MOF patients had not received any transfusions and 14 had received at least 1 RBC unit. Ten of the MOF patients were not taken along in the GE study, for various logistic reasons. 14 matched control patients were selected, based on similar age, gender, the number of RBC units within 24 hours after surgery, type and duration of surgery. Randomisation group was no selection criterion, because at time of the first selection of 29 patients, the blinded RCT was still ongoing.

In order to be eligible for the transfusion groups, a patient should have received at least one RBC unit during or within the first 24 hours after surgery. To be selected for the non-transfusion group, patients should not have had any blood products at all up to 24 hours after surgery.
The patients from groups i and ii were non-transfused (N=15), and groups iii and iv were transfused (N=14). All RBC units were pre storage leukocyte reduced and transfused according to standard hospital protocol. Groups i and iii did not develop MOF (N=14) and groups 2 and 4 would develop MOF (N=15).

After confirmation of the expression-array outcome regarding individual ΔGE of three candidate relevant genes by RT PCR, 21 pre- and post surgical RNA-samples from intermediate risk cardiac surgery procedures, including two patients who developed MOF, were selected from the 152 patients matching the previous 29 case-control patients.

### Sample collection and processing

Whole blood samples were withdrawn by vena-puncture prior to anaesthesia and surgery and at day 1 after surgery (24 hours after CPB) and were kept at 4°C until RNA stabilisation. The RNA of 2 aliquots of 500 microliters whole blood was stabilised at day 1 (0 -2) with RNALater (Applied Biosystems/Ambion, Austin, TX USA). RNA extraction was performed with the RiboPure™-blood kit (Applied Biosystems/Ambion, Austin, TX USA), according to standard protocol including the optional DNase treatment. RNA quantity was determined with a photospectrometer (Nanodrop 1000). Additionally, good RNA integrity was verified for a random selection of samples with the bioanalyzer (Agilent Technologies, Santa Clara, CA USA).

From each of the four patient groups, 50 ng blood RNA from each of the individual pre surgical samples and 50 ng blood RNA from each of the individual post surgical samples were pooled successively, before applying on the micro-array.

### Expression arrays

The Quality control, RNA labeling, hybridisation and data extraction were performed at ServiceXS (Leiden, The Netherlands). RNA concentration was measured using a Nanodrop ND-1000 spectrophotometer (Nanodrop Technologies, Wilmington, DE, U.S.A). The RNA quality and integrity was determined using Lab-on-Chip analysis on an Agilent 2100 Bioanalyzer (Agilent Technologies, Inc., Santa Clara, CA, U.S.A.). Biotinylated cRNA was prepared using the Illumina TotalPrep RNA Amplification Kit (Ambion, Inc., Austin, TX, U.S.A.) according to the manufacturer's specifications (www.illumina.com) starting with 500 ng total RNA. Experiments were executed by the Certified Illumina Service Provider ServiceXS (www.Servicexs.com). Briefly, per sample, 750 ng of cRNA was used to hybridise to the Sentrix HumanRef-8 v3 Expression BeadChips (Illumina, Inc., San Diego, CA, U.S.A.). Each BeadChip contains twelve arrays. Hybridisation and washing were performed on eight arrays according to the Illumina standard assay procedure. Scanning was performed on the Illumina iScanner (Illumina, Inc., San Diego, CA, U.S.A.). The GenomeStudio was used to extract de Illumina signal intensities. Normalisation of spot intensities was performed per array using GeneSpring software by quantile normalisation. Definitive quantile normalization made the distribution of expression values of all samples in the experiment the same. Normalised GE values were expressed successively as fold changes.

### RT-PCR

Based upon differences of ΔGE levels between the patient groups, a selection of 3 genes was made for confirmation with 2-step reverse transcriptase Real-Time PCR. Assays were ordered from Applied Biosystems (Assay ID's: Hs00968978-m1 (ARG1); Hs00376118-m1 (zinc finger DHHC type containing19 (ZDHHC19); Hs00957562_m1 (MMP9); Hs00174759_m1 (IL-1R2); Hs00187256_m1 (IL18RAP)).

From all patient samples, cDNA was constructed from the same RNA pool as used on the arrays. Reverse transcriptase was performed with the SuperScript® II Reverse Transcriptase using random primers (Invitrogen Corporation, Carlsbad, CA USA).

The 7500 Fast RT-PCR (Applied Biosystems Inc., Foster City, CA USA) was used on standard modus and standard program with the Gene expression master mix (Applied Biosystems Inc., Foster City, CA USA). In each run, all samples, including a reference sample, consisting of pooled cDNA of 5 volunteer blood donors, was taken along in 4-fold. Outliers were removed, before analysis, when identified in the quarto RT PCR measures by the Grubbs'test (using critical Z for n=4), or named ESD (extreme studentized deviate) method
(http://www.graphpad.com/quickcales/GrubbsHowTo.cfm). All RT-PCR output was corrected for its reference sample in the same run. For all patients, ΔGE levels were calculated. RT-PCR data were analysed statistically with SPSS version 15.0 for windows (SPSS Inc., Chicago,IL,USA). The ΔGE 24 hrs after surgery with and without transfusion were compared with the ΔGE in MOF and no MOF patients. GE levels before- and after surgery were compared with blood samples from MOF and no MOG groups, therefore paired Wilcoxon tests were performed. We considered a p-value ≤ 0.05 statistically significant. Data are expressed as medians with interquartile ranges (IQR).

### Data analysis

Regarding the analysis of micro-array data using pooled RNA we systematically performed the following steps: First, transcriptome responses, quantified for up- and downregulated genes, were selected based on > 2 fold change in ΔGE between pre- surgery pooled RNA, and pooled RNA samples from each patient group and ranked on order of top magnitude fold changes. Second, for each above selected up- and downregulated gene, transcriptome responses were calculated and selected by the difference > 2 fold change in ΔGE between the groups. Third, difference in ΔGE between group i and group iii was defined as "the transfusion effect" on transcriptome response. Difference in ΔGE between group i and group ii was entitled: "the MOF effect" on transcriptome response and the difference in ΔGE between group i and group iv was defined as "the BT in MOF effect" on transcriptome response (Figure 1). Fourth, each quantified difference between the "surgery"-, "MOF"- and BT in MOF" effects were ranked on those transcriptomes revealing the highest "MOF-effect", BT-effect and "BT in MOF" effect on top and images were constructed using matrix2png 1.0.7 (Grisoni ML, et al. J Biol Chem 1999; 274: 21491-21494).

The observed increase in ΔGE of ARG1, ZDHHC19, IL-1R2, MMP9 and IL18RAP transcripts on the expression arrays were individually confirmed by pre-and post-surgery blood RNA samples from the 29 patients of the four groups by RT PCR. Confirmation by RT PCR of the differences in fold changes between the groups of these genes were chosen based on i) the highest MOF-effects (on transcriptome response between the "MOF" and "surgery" group, on top: MMP9, ARG1 and ZDHHC19) and the top ΔGE difference of the gene known to associated with cardiovascular risk: IL18RAP. IL-1R2 gene was chosen based on the highest "BT in MOF" effect.

Successively, the specificity and sensitivity of MOF-prediction were assessed by ΔGE in an additional selection of intermediate risk cardiac surgery patients as confirmation cohort (n=21) for those transcripts which revealed non parametric significances (p≤ 0.05) for ZDHHC19, MMP9- and IL18RAP and showed the optimal combination and range of ΔGE for identification of all MOF patients in the prior case control patients (n=29).

### Statistics

All analyses were conducted using SPSS version 15.0 for windows (SPSS Inc., Chicago, IL USA). To test the difference between paired pre- and postoperative GE values, Wilcoxon tests were performed using the RT-PCR data. Mann-Whitney U tests were performed for comparisons between the MOF-group with the no MOF-group. Additionally for the RT-PCR data, Mann-Whitney U tests were performed to test the difference in ΔGE between transfused and non-transfused patients and between non-MOF and MOF patients. P-values ≤ 0.05 were considered significant. Data are expressed as medians with interquartile ranges (IQR).

### Results

### Patients

Patient demographics from the total case-control of 50 patients are shown in Table 2 and divided in the peri-operative characteristics of 17 MOF "case" patients and 33 matched controlled no MOF intermediate risk cardiac surgery patients. No significant differences in patient variables were detected between the both case and control, except for duration of ICU stay and ventilation support, which both were lower in no MOF patients. The MOF patients tended to receive more autologous blood by cell saver, but the nested control was matched and selected as much as possible on the number of administered blood products.

### Genome wide screening on transcriptome responses after surgery

Obtained from micro expression array on the pooled blood RNA, 1047 genes revealed >2 fold ΔGE (453 up and 594 down regulated transcriptomes) after surgery in at least one of the 4 groups. The 30 most pronounced differences are ranked and listed in Figure 1 for those patients that either did or did not develop MOF.

### Transcriptome responses by ΔGE in MOF and no MOF patients (group ii versus group i)

13 genes showed >2 fold difference in upregulation in the open reading frame 59 on chromosome19 (C19orf59: +7 fold); matrix metallopeptidase 9 (MMP9: +7 fold), arginase-1 (ARG1: +6 fold), interleukin-1 receptor type 2 (IL-1R2: +5 fold), Zn²⁺-finger protein ZDHHC19 (+5 fold), glycolyse enzyme PFKFB3 (+4 fold), Interleukin-18 receptor associated protein (IL18RAP: + 4 fold), Ca²⁺ binding protein S100A12 (+4 fold), complement decay-accelerating factor CD55 (+2 fold), FK506 binding protein 5 (FKBP5: +2 fold), Carbonic anhydrase 4 (CA4:+2 fold) and Alkaline phosphatase (ALPL: +2 fold). The by surgery down regulated transcriptome encoding for myxovirus (influenza virus) resistance 1 (MX1) protein was +2 fold up regulated revealing however no difference in ΔGE in MOF (Figure 1).

From the down regulated transcripts, three proteins encoding for the eosinophilic lipase CLC (:-6 fold), prostaglandin-endoperoxide synthase 2, also known as cyclooxygenase-2 (PTGS2: -3 fold) and interleukin 8 (IL-8: -2 fold) were more down regulated in the MOF group (Figure 1).

### Selection for RT-PCR confirmation

From the 1047 genes showing altered expression between pre- and post-surgical levels, the top 5 transcriptomes revealing the largest differences in ΔGE fold increases between either group ii and i were chosen for confirmation with RT-PCR, based on functional relevance. RT PCR for C19orf59 was not applied, because of it's unknown biological function. MMP9-, Arginase 1- and ZDHHC19 RT PCR were selected, representing genes with a known biological function possibly distinguishing MOF and no MOF patients.

### RT-PCR

The RT-PCR assays could confirm the increases of ΔGE fold changes of all the 5 chosen transcripts before and after surgery which was observed in pooled RNA on the expression array. Because the individual paired ΔGE's of the transcipts did not reflect the normal, but a nonparametric distribution, we used nonparametric testing to demonstrate for each transcript whether or not the median fold increase did meet the criteria of significance between MOF and no MOF patients (Table 3). The median ΔGE of ZDHHC19, MMP9 and IL18RAP transcripts showed to be significant increased in MOF patients, as compared to no MOF patients, when compared between before and after surgery: 20,4 (10,1-34,6) for ZDHHC, 8,6 (2,3-14,5) for IL18RAP, 7,9 (4,1-26,7) for MMP9, 11,7 (7,9-41,0) for ARG1 and 7,1 (3,1-21,0) fold increase for IL-1R2, whereas MOF patients showed significant/marked increases of median ZDHHC19 of: 75,3 (42,0-251,7; p=0.002), IL18RAP: 20,6 (8,5-27,1; p=0.051) and of MMP9: 23,2 (14,8-31,8; p=0,033), respectively (Table 3). One IL-1R2 detection failed technically in the samples from one MOF patient without BT. The median fold changes of ARG1 and IL1R2 transcripts in MOF-patients were not significantly increased after surgery as compared to non-MOF patients and were further therefore excluded for analysis in the search of combinations transcript and ΔGE responses to predict MOF. However, the median differences of 4 of the 5 transcripts between the four subgroups were not significant, probably due to the small sample sizes. Only a significant increase in ZDHHC19 GE in the transfused MOF patient subgroup iv as compared to the group i (p=0.021), could reveal a possible effect of RBC transfusion in only MOF patients on ZDHHC19 GE, because no significant differences in ZDHHC19 GE response between groups i and the other groups ii and iii were shown.

### MOF prediction

The optimal cut-offs in fold changes were chosen to identify the most true (case) MOF patients and less true (control) no MOF patients: Using the PCR data of the optimal fold change outcomes after surgery for MOF prediction were calculated by using a quantitative combination of three transcripts, because none of the individual ΔGE increases of candidate genes MMP9, ZDHHC19 and IL18RAP alone or paired did predict MOF with > 80% sensitivity (Table 4). The combination of ΔGE of the two genes MMP9 >14.8 fold and IL18RAP> 8,8 fold increase revealed no better sensitivity (71%) and specificity (67%) in MOF prediction (Table 4). However, this combination, or MMP9 transcript responses >14,8 fold together with 45<ΔGE<93 fold increase by ZDHHC19, or >93 fold increase alone in ZDHHC19 transcripts, increased the maximum sensitivity up to 100% (Table 5). In the confirmation cohort of 21 intermediate risk cardiac surgery procedures these optimal combinations of three GE biomarkers and their cut-offs could recognise all (2) MOF patients with a specificity of 58% and sensitivity of 100% (Table 6). The observed decrease in specificity in the confirmation cohort is not surprising, because of the small confirmation cohort, the three transcripts were selected by expression array on (case) MOF and not on (control) no MOF.

In the total cohort of 50 patients the comparison between KNAUS and an optimal GE response in MOF and no MOF patients identified 48 hrs after surgery 73% vs 79% specificity, respectively, in favour of GE responses which identified 100% MOF using optimal cut-offs in fold changes by: 93<ZDHHC19<269; 23,5<IL18RAP<36; 14,8<MMP9<26,1 (Table 7). The sensitivity by cumulative KNAUS 24 and 48 hr after surgery > 4 was 71%, whereas the optimal GE signature of the three genes was associated with all patients who developed MOF (100% sensitivity).

**TABLE 2**

| Case control (n=29) & confirmation cohort (n=21) | SIRS patients | MOF patients | P value |
|---|---|---|---|
| Characteristics of the intermediate risk patients | (n = 33) | (n = 17) | |
| | | | |
| male/female (n) | 31/2 | 14/3 | 0.196 |
| age (years) | 66 ± 8 | 66 ± 10 | 0.936 |
| BMI (kg/m2) | 27.2 ± 3.7 | 27.7 ± 3.5 | 0.677 |
| EuroSCORE (%) | 2.9 ± 1.6 | 3.8 ± 2.8 | 0.25 |
| Diabetes (n) | | | 0.752 |
| type 2 | 4 | 3 | |
| type 1 | 1 | 1 | |
| Left ventricular functioning | | | 0.385 |
| moderate | 8 | 7 | |
| poor | 1 | 0 | |
| Preoperative Hb (mmol/L) | 9.3 ± 0.8 | 9.2 ± 0.4 | 0.785 |
| Cockroft renal clearance (mL/min) | 87 ± 23 | 88 ± 30 | 0.87 |
| Type of surgery | | | |
| CABG only | 21 | 6 | 0.108 |
| Valvular surgery only | 6 | 7 | 0.157 |
| Combination valve & CABG | 6 | 4 | 0.941 |
| operation time (minutes) | 314 ± 66 | 346 ± 120 | 0.322 |
| Perfusion time (minutes) | 160 ± 34 | 185 ± 76 | 0.205 |
| Cross clamp time (minutes) | 111 ± 25 | 135 ± 64 | 0.156 |
| *Cell saver blood returned (mL) | 0(425) | 230 (670) | 0.053 |
| Tranexamic acid administered (n) | 8 | 5 | 0.654 |
| Aprotinin administered (n) | 12 | 4 | 0.465 |
| Steroids administered (n) | 8 | 4 | 0.959 |
| ICU stay duration (days) | 1.4 ± 0.7 | 9.3 ± 13.9 | 0.032 |
| *Ventilatory support (hours) | 8 (6) | 18 (146) | 0.055 |
| 24 Hour MCTD blood loss (mL) | 930 ± 507 | 784 ± 413 | 0.312 |
| *PRBC transfused < 24 hours (units 240 mL) | 0(2) | 1 (2) | 0.338 |
| *FFP transfused < 24 hours(units 240 mL) | 0(2) | 0 (2) | 0.425 |
| *Thrombocytes transfused < 24 hours(units 170 mL) | 0(0) | 0 (1) | 0.236 |
| Highest cardiac index < 24 hours (1/min/m²) | 4.2 ± 0.7 | 3.7 ± 0.8 | 0.236 |
| Use of IABP postoperatively (n) | 0 | 2 | 0.212 |
| *Highest noradrenalin < 24 hours (Y) | 0(0) | 0.10 (0) | 0.082 |
| Myocardial infarction (n) | 1 | 2 | 0.546 |
| Highest leukocyte count < 24 hours (x 10⁹/L) | 17.2 ± 13.1 | 12.9 ± 2.6 | |
| Rethroacotomy performed < 24h (n) | 0 | 1 | 0.213 |
| IABP used (n) | 0 | 2 | 0.212 |
| CVVH used (n) | 0 | 2 | 0.212 |

**TABLE 3:**

| | **Case-control: n=29** | | | |
|---|---|---|---|---|
| **Table 3** | | | | |
| **gene** | **Clinical outcome** | **N** | **Fold changes (IQR)** | **P (two sided)** |
| ZDHHC19 | MOF | 14 | 75,3 (42,0 -251,7) | 0,002 |
| | no MOF | 14 | 20,4 (10,1 - 34,6) | |
| MMP9 | MOF | 14 | 23,2 (14,8 - 31,8) | 0,033 |
| | no MOF | 14 | 7,9 (4,1 - 26,7) | |
| IL18RAP | MOF | 15 | 20,6 (8,5 - 27,1) | 0,051 |
| | no MOF | 14 | 8,6 (2,3 - 14,5) | |
| IL1R2 | MOF | 14 | 19, (5,6 - 46,3) | 0,081 |
| | no MOF | 14 | 7,1 (3,1 - 21,0) | |
| ARG1 | MOF | 15 | 28,5 (8,4 - 97,9) | 0,312 |
| | no MOF | 14 | 11,7 (7,9 - 41,0) | |

Median ΔGE levels in MOF and no MOF of the top 5 functional transcriptome upregulation after surgery as measured with RT-PCR in the case control (n=29). Two sided p-values after Mann-Whiney-U testing demonstrated non parametric significant increase in median ΔGE between both groups in three transcripts. Optimal combinations and ranges in individual ΔGE of these transcripts were chosen to predict MOF.

**Table 4:**

| | **nested case-control fold changes** | | |
|---|---|---|---|
| **TABLE 4** | | MOF | no MOF |
| **gene** | | **Case** | **Control** |
| ZDHHC19 | >93 | 6 | 1 |
| | 45-93$ | 5 | 1 |
| | <45 (no) | 4 | 12 |
| MMP9 | >14,8^ | 12 | 4 |
| | <14,8 (no) | 3 | 10 |
| IL18RAP | >8,8* | 11 | 5 |
| | <8,8 | 4 | 9 |
| combinations | (*and ^) | 10 | 4 |
| | no | 5 | 10 |
| | ($ and ^) | 5 | 1 |
| | no | 10 | 13 |
| | ($ and *) | 3 | 1 |
| | no | 12 | 13 |

Incidences of individual fold changes of ZDHHC19, MMP9 and IL18RAP transcripts and in combination of two types of transcripts after surgery in 29 patients (case control) developing MOF or no MOF (SIRS). The cut-offs in fold changes were chosen to identify the most true (case) MOF patients and less true (control) no MOF patients.

**Table 5**

| TABLE 5 | Case | Control |
|---|---|---|
| nested case-control | | |
| n=29 | MOF | no MOF |
| profile + | 15 | 3 |
| profile - | 0 | 11 |

Incidences of a chosen combined ΔGE profile with 100% sensitivity for MOF as compared to patients who don't meet this exclusive ΔGE profile in this case control (n=29). Corresponding specificity= 3/(11+3)=79%.

Profile += ΔGE ZDHHC19 >93, or IL18RAP>8,8 and MMP9 >14,8, or MMP9 >14,8 and 93>ZDHHC19>45 combination was chosen to identify all MOF patients. Profile - = ΔGE ZDHHC19 <45, MMP9<14,8 and IL18RAP<8,8.

**Table 6**

| Table 6 | Case | Control |
|---|---|---|
| confirmation cohort | | |
| n=21 | MOF | no MOF |
| profile + | 2 | 8 |
| profile - | 0 | 11 |
| | | |

Confirmation cohort of 21 medium risk cardiac surgery patients using the chosen ΔGE profile for prediction of developing postoperative MOF reveals 100% sensitivity and 58% specificity. Profile += ΔGE ZDHHC19 >93, or IL18RAP >8,8 and MMP9 >14,8, or MMP9 >14,8 and 93>ZDHHC19>45. Profile - = ΔGE ZDHHC19 <45, MMP9<14,8 and IL18RAP<8,8.

**Table 7**

| Table 7 | case | control | sens | spec |
|---|---|---|---|---|
| profile | | | | |
| optimum* | 17 | 7 | 1,00 | 0,79 |
| profile - | 0 | 26 | | |

| cum KNAUS | case | control | sens | spec |
|---|---|---|---|---|
| >4 | 12 | 9 | 0,70 | 0,73 |
| <5 | 5 | 24 | | |

In the total cohort of 50 patients developing MOF (case) or no MOF (control: SIRS) were shown by the incidences of individual fold changes of ZDHHC19, MMP9 and IL18RAP transcripts and cumulative KNAUS scores at 24 and 48 hours after surgery. Cumulative KNAUS >4 vs optimal GE response cut-offs in MOF and no MOF patients were compared 48 hrs after surgery. *The cut-offs in fold changes were optimised using a GE Profile identifying all MOF patients and most true no MOF patients expressing fold changes of either 93<ZDHHC19<269, 23,5<IL18RAP<36, or 14,8<MMP9<26.1.

## Claims

1. A method for typing a sample of an individual to determine a risk of said individual to develop multiple organ failure, comprising:
- determining a level of expression of a product of at least one gene selected from Table 1, said level of expression being determined using said sample of said individual;
- comparing said level of expression with a level of expression of a product of said at least one gene of a reference sample; and
- typing said sample based on the levels of expression determined for said at least one gene.

2. A method according to claim 1, comprising determining a level of expression of a product of at least two genes selected from Table 1, wherein at least one of said genes is MMP9 or IL18RAP.

3. A method according to claim 1 or 2, wherein said individual has undergone surgery.

4. A method according to any one of the preceding claims, wherein said sample is from an individual after surgery and said reference sample is from said individual prior to surgery.

5. A method according to any one of the preceding claims, wherein said at least two genes are selected from the group consisting of ZDHHC19, MMP9 and IL18RAP.

6. A method according to any one of the preceding claims, comprising determining whether a fold change in the level of expression of ZDHHC19 in said sample of said individual is at least 42 as compared to the level of expression of ZDHHC19 in said reference sample, and/or whether a fold change in the level of expression of IL18RAP in said sample of said individual is at least 8.5 as compared the level of expression of IL18RAP in said reference sample, and/or whether a fold change in the level of expression of MMP9 in said sample of said individual is at least 14.8 as compared to the level of expression of MMP9 in said reference sample, which is indicative of the presence of a risk of said individual to develop multiple organ failure.

7. A method according any one of the preceding claims, comprising determining whether a fold change in the level of expression of ZDHHC19 in said sample of said individual is between 42 and 300 as compared to the level of expression of ZDHHC19 in said reference sample, and/or a fold change in the level of expression of IL18RAP in said sample of said individual is between 8.5 and 60 as compared the level of expression of IL18RAP in said reference sample, and/or a fold change in the level of expression of MMP9 in said sample of said individual is between 14.8 and 50 as compared to the level of expression of MMP9 in said reference sample, which is indicative of the presence of a risk of said individual to develop multiple organ failure.

8. A method according to any one of claims 1-3, wherein said individual is suffering from a condition or is receiving or has received treatment associated with a risk of developing multiple organ failure, and wherein said reference sample comprises pooled samples of individuals suffering from the same condition or having received the same treatment who did not develop multiple organ failure as a result of said condition.

9. A method according to any one of the preceding claims, comprising determining a level of expression of a product of at least five genes selected from Table 1.

10. A method according to any one of the preceding claims, wherein said sample of an individual is a blood sample.

11. A method according to any one of the preceding claims, wherein said product is RNA.

12. A method according to any one of the preceding claims, wherein said surgery is cardiac surgery.

13. A kit of parts comprising:
- at least two gene-specific primers selected from the group consisting of a ZDHHC19-specific primer, an MMP9-specific primer and an IL18RAP-specific primer, and/or
- at least two gene-specific probes selected from the group consisting of a ZDHHC19-specific probe, an MMP9-specific probe and an IL18RAP-specific probe.

14. Use of an MMP9-specific primer or an IL18RAP-specific primer, and/or a use of an MMP9-specific probe or an IL18RAP-specific probe for typing a sample of an individual to determine a risk of said individual to develop multiple organ failure.

15. Use of a kit of parts according to claim 13 for typing a sample of an individual to determine a risk of said individual to develop multiple organ failure.
